# EUROPEAN PATENT APPLICATION

(11) **EP 2 014 647 A1**
(43) Date of publication of application: **14.01.2009**
(21) Application number: 06767862.3
(22) Date of filing: 28.06.2006
(51) Int. Cl.: C07D 209/52, C07D 209/48

(54) **PROCESS FOR PRODUCING POLYCYCLIC LACTAM**

(30) Priority: 17.04.2006 JP 2006113073
(71) Applicant: Sumitomo Chemical Company, Limited, Tokyo 104-8260 (JP)
(72) Inventor: HIRATA, Norihiko, Suita-shi, Osaka 565-0836 (JP); UEMURA, Toshitsugi, Toyonaka-shi, Osaka 561-0802 (JP); USHIO, Hideki, Ibaraki-shi, Osaka 567-0841 (JP)
(74) Representative: Duckworth, Timothy John
(86) International application number: PCT/JP2006/313354
(87) International publication number: WO 2007/122743

(57) **Abstract**

1. A method of producing lactams of the following formula (1) comprising the following three steps A to C: 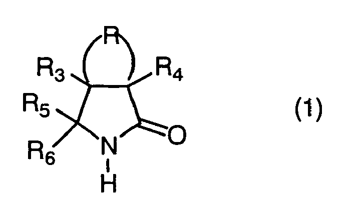 [when R₃ and R₄ are both a hydrogen atom, R represents a substituted methylene group or an optionally substituted polymethylene group, when at least one of R₃ and R₄ is a substituent other than a hydrogen atom, R represents an optionally substituted methylene group or an optionally substituted polymethylene group, R₃, R₄, R₅ and R₆ represent each independently a hydrogen atom, halogen atom, cyano group, optionally substituted alkyl group, optionally substituted alkenyl group, optionally substituted aryl group, optionally substituted amino group, -ORₐ group, -SR_{b} group or - COOR_{c} group.]
(Step A)
a step of reacting lactones of the following formula (2): 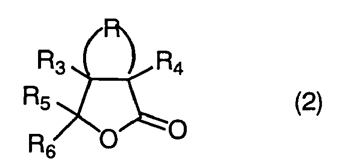 and imides of any of the following formulae (3a), (3b) and (3c): 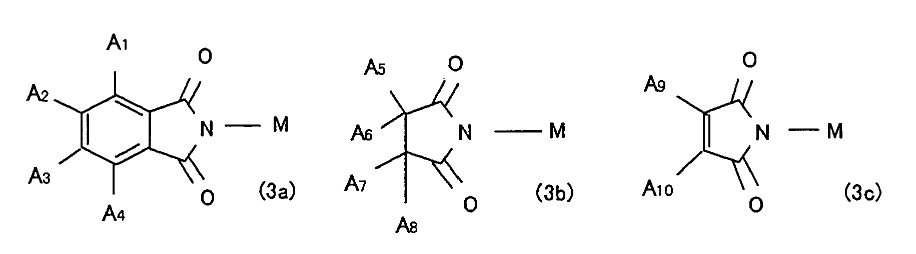 [A₁, A₂, A₃, A₄, A₅, A₆, A₇, A₈, A₉ and A₁₀ represent each independently a hydrogen atom, halogen atom, nitro group, alkyl group, alkenyl group, optionally substituted aryl group, optionally substituted acyl group or -OR_{d}.] to produce the correspondent carboxylic acids,
(Step B)
a step of esterifying the carboxylic acids to produce the correspondent esters,
(Step C)
a step of reacting the esters and amines to produce lactams of the formula (1).

## Description

### Technical Field

The present invention relates to a method of producing polycyclic lactams.

### Background Art

As a method of producing polycyclic lactams, there is known a method of reacting lactones, 3-oxabicyclo[3.1.0]hexan-2-one with phthalimide potassium in an N,N-dimethylformamide solvent, then, esterifying using an acid carried on a resin, subsequently, performing N-deprotection reaction using an amine compound carried on a resin (see, Synlett, 10, 1641(2002)), however, this method has a problem from the standpoint of cost because of necessity of use of a special reagent carried on a resin for the reaction of lactones and phthalimide potassium, and is unsatisfactory also in the yield of polycyclic lactams.

The present inventors have investigated an industrially advantageous method of producing polycyclic lactams, and resultantly found that polycyclic lactams can be produced with good yield by using a certain kind of polycyclic lactones as a raw material.

### Disclosure of the Invention

The present invention has an object of providing a method for producing polycyclic lactams with good yield.

That is, the present invention provides the following [1] to [23].
[1]. A method of producing lactams of the following formula (1) comprising the following three steps A to C: 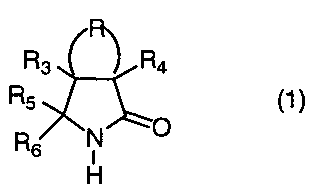 [in the formula (1), when R₃ and R₄ are both a hydrogen atom, R represents a substituted methylene group or an optionally substituted polymethylene group having 2 to 4 carbon atoms, when at least one of R₃ and R₄ is a substituent other than a hydrogen atom, R represents an optionally substituted methylene group or an optionally substituted polymethylene group having 2 to 4 carbon atoms, and one or no-mutually-adjacent two methylene groups constituting the polymethylene group may be substituted by an oxygen atom, one or two ethylene groups constituting the polymethylene group may be substituted by a vinylene group, no-mutually-adjacent two methylene groups constituting the polymethylene group may be mutually connected via an oxygen atom, methylene group, ethylene group or vinylene group,
   R₃, R₄, R₅ and R₆ represent each independently a hydrogen atom, halogen atom, cyano group, optionally substituted alkyl group having 1 to 10 carbon atoms, optionally substituted alkenyl group having 2 to 10 carbon atoms, optionally substituted aryl group having 6 to 20 carbon atoms, optionally substituted amino group, -ORₐ group, -SR_{b} group or -COOR_{c} group, Rₐ and R_{b} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms, and R_{c} represents a hydrogen atom, alkyl group having 1 to 10 carbon atoms or aralkyl group having 7 to 20 carbon atoms.]
   (Step A)
      a step of reacting lactones of the following formula (2): 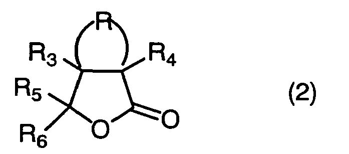 [in the formula (2), R and R₃ to R₆ represent the same meanings as described above.]
      and imides of any of the following formulae (3a), (3b) and (3c): 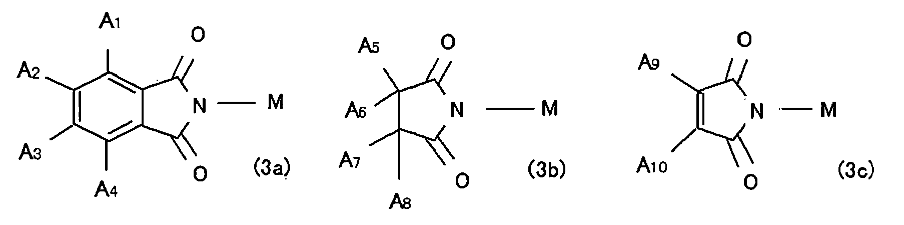 [in the formulae (3a), (3b) and (3c), A₁, A₂, A₃, A₄, A₅, A₆, A₇, A₈, A₉ and A₁₀ represent each independently a hydrogen atom, halogen atom, nitro group, alkyl group having 1 to 6 carbon atoms, alkenyl group having 2 to 4 carbon atoms, optionally substituted aryl group having 6 to 20 carbon atoms, optionally substituted acyl group having 2 to 4 carbon atoms or -OR_{d}, R_{d} represents a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms, and M represents a hydrogen atom or alkali metal atom.]
      to produce the correspondent carboxylic acids of the following formulae (4a), (4b) and (4c): 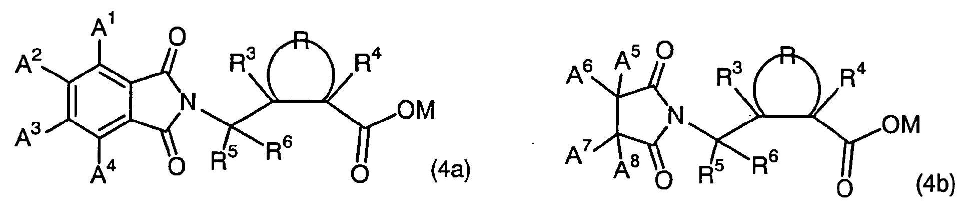 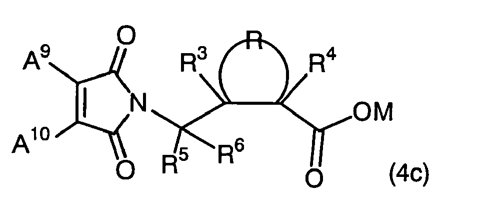 [in the formulae (4a), (4b) and (4c), R, R₃, R₄, R₅, R₆, A₁, A₂, A₃, A₄, A₅, A₆, A₇, A₈, A₉, A₁₀ and M represent the same meanings as described above.]
   (Step B)
      a step of esterifying the carboxylic acids of any of the formulae (4a), (4b) and (4c) obtained in the step A to produce the correspondent esters of the following formulae (5a), (5b) and (5c): 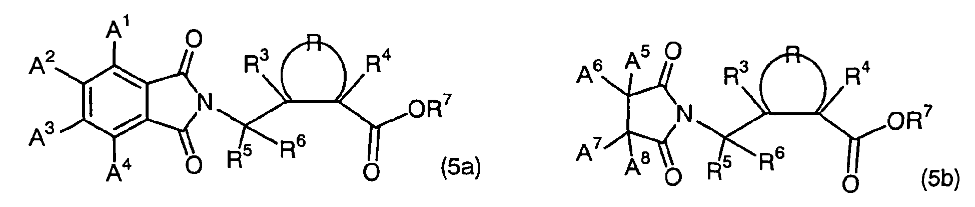 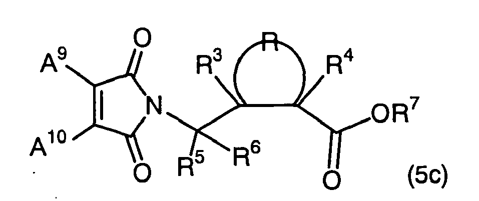 [in the formulae (5a), (5b) and (5c), R, R₃, R₄, R₅, R₆, A₁, A₂, A₃, A₄, A₅, A₆, A₇, A₈, A₉ and A₁₀ represent the same meanings as described above, and R₇ represents an alkyl group having 1 to 10 carbon atoms, alkenyl group having 2 to 10 carbon atoms or aralkyl group having 7 to 20 carbon atoms.]
   (Step C)
      a step of reacting the esters of any of the formulae (5a), (5b) and (5c) obtained in the step B and amines to produce lactams of the formula (1).
[2]. The production method according to [1] wherein the lactones of the formula (2) and the imides of any of the formulae (3a), (3b) and (3c) are reacted in an amide solvent of the following formula (6): 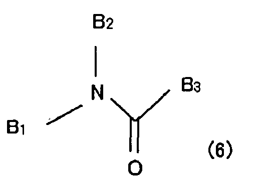 [in the formula (6), B₁ and B₂ represent each independently a hydrogen atom or alkyl group having 1 to 5 carbon atoms, B₃ represents a hydrogen atom, alkyl group having 1 to 10 carbon atoms or aryl group having 6 to 20 carbon atoms, and B₂ and B₃ may be connected to form a cyclic structure together with an atom to which they are connected.].
[3]. The production method according to [2] wherein the amide solvent of the formula (6) is N,N-dimethylacetamide.
[4]. The production method according to any one of [1] to [3] wherein the imides are imides of the formula (3a).
[5]. The production method according to any one of [1] to [4] wherein the imides of the formula (3a) are phthalimide potassium.
[6]. The production method according to any one of [1] to [5] wherein the carboxylic acids of any of the formulae (4a), (4b) and (4c) and alcohols of the following formula (7)

   R₇-OH (7)

   (wherein, R₇ represents an alkyl group having 1 to 10 carbon atoms, alkenyl group having 2 to 10 carbon atoms or aralkyl group having 7 to 20 carbon atoms.) are reacted to produce esters of any of the formulae (5a), (5b) and (5c), and the resultant esters of any of the formulae (5a), (5b) and (5c) and amines of the following formula (8):

   R₈-NH₂ (8)

   (wherein, R₈ represents an optionally substituted amino group or alkyl group having 1 to 5 carbon atoms optionally substituted with an amino group.) are reacted to produce lactams of the formula (1).
[7]. The production method according to any one of [1] to [6] wherein R₅ and R₆ represent a hydrogen atom.
[8]. The production method according to any one of [1] to [7] wherein R₃ and R₄ represent a hydrogen atom.
[9]. The production method according to any one of [1] to [8] wherein R is a group selected from the following group:
   -(CH₂)₂-, -(CH₂)₃-, -(CH₂)₄-, (CH₃)₂C<, (C1)₂C<, (F)₂C<, >CH(CO₂C₂H₅), 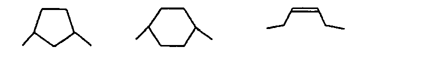 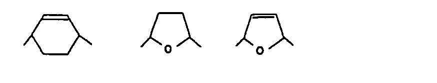 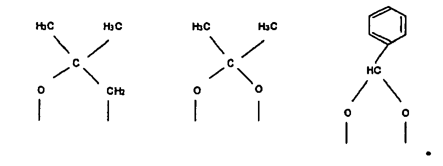
[10]. The production method according to any one of [1] to [9] wherein R is a group selected from the following group:
   -(CH₂)₂-, -(CH₂)₃-, -(CH₂)₄-, (CH₃)₂C<.
[11]. The production method according to any one of [1] to [10] wherein R is a (CH₃)₂C< group.
[12]. Carboxylic acids of the following formula (4a): 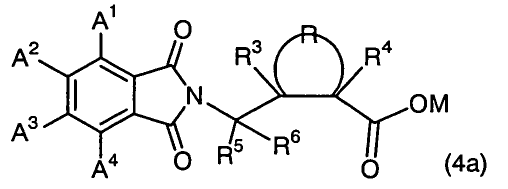 [in the formula (4a), R, R₃ to R₆, A₁ to A₄ and M represent the same meanings as described above.].
[13]. The carboxylic acids according to [12] wherein, in the formula (4a), R represents a group selected from the group consisting of -(CH₂)₂-, - (CH₂)₃-, -(CH₂)₄- and (CH₃)₂C<, and R₃, R₄, R₅, R₆, A₁, A₂, A₃ and A₄ represent a hydrogen atom.
[14]. 2,2-dimethyl-3-[(1,3-dihydro-1,3-dioxo-2H-isoindol-2-yl)methyl]-cyclopropanecarboxylic acid.
[15]. A potassium salt of 2,2-dimethyl-3-[(1,3-dihydro-1,3-dioxo-2H-isoindol-2-yl)methyl]-cyclopropanecarboxylic acid.
[16]. (1R,3S)-2,2-dimethyl-3-[(1,3-dihydro-1,3-dioxo-2H-isoindol-2-yl)methyl]-cyclopropanecarboxylic acid.
[17]. A potassium salt of (1R,3S)-2,2-dimethyl-3-[(1,3-dihydro-1,3-dioxo-2H-isoindol-2-yl)methyl]-cyclopropanecarboxylic acid.
[18]. Esters of the following formula (5a): 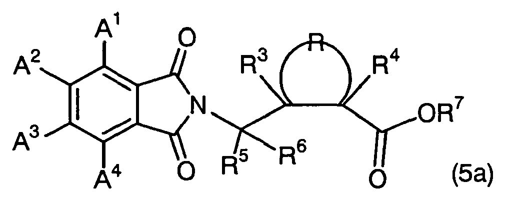 [in the formula (5a), R, R₃ to R₇ and A₁ to A₄ represent the same meanings as described above.].
[19]. The esters according to [18] wherein, in the formula (5a), R represents a group selected from the group consisting of -(CH₂)₂-, -(CH₂)₃-, -(CH₂)₄- and (CH₃)₂C<, and R₃, R₄, R₅, R₆, A₁, A₂, A₃ and A₄ represent a hydrogen atom.
[20]. The esters according to [18] or [19] wherein R₇ in the formula (5a) is an alkyl group having 1 to 4 carbon atoms.
[21]. 2,2-dimethyl-3-[(1,3-dihydro-1,3-dioxo-2H-isoindol-2-yl)methyl]-cyclopropanecarboxylates.
[22]. Methyl 2,2-dimethyl-3-[(1,3-dihydro-1,3-dioxo-2H-isoindol-2-yl)methyl]-cyclopropanecarboxylate.
[23]. Methyl (1R,3S)-2,2-dimethyl-3-[(1,3-dihydro-1,3-dioxo-2H-isoindol-2-yl)methyl]-cyclopropanecarboxylate.

### Modes for Carrying Out the Invention

The present invention will be illustrated in detail below.

The step A is a step of reacting lactones of the above-described formula (2) [hereinafter, abbreviated as lactones (2) in some cases] and imides of any of the above-described formulae (3a), (3b) and (3c) to produce the correspondent carboxylic acids of the above-described formulae (4a), (4b) and (4c) [hereinafter, abbreviated as carboxylic acids (4a), (4b) or (4c), respectively, in some cases].

In the lactones (2) to be used in the present invention, when R₃ and R₄ described later are both a hydrogen atom, R represents a substituted methylene group or an optionally substituted polymethylene group having 2 to 4 carbon atoms, and when at least one of R₃ and R₄ is a substituent other than a hydrogen atom, R represents an optionally substituted methylene group or an optionally substituted polymethylene group having 2 to 4 carbon atoms. One or no-mutually-adjacent two methylene groups constituting the polymethylene group may be substituted by an oxygen atom, and one or two ethylene groups constituting the polymethylene group may be substituted by a vinylene group. Further, no-mutually-adjacent two methylene groups constituting the polymethylene group may be mutually connected via an oxygen atom, methylene group, ethylene group or vinylene group.

The substituent optionally substituted on the above-described methylene group or polymethylene group having 2 to 4 carbon atoms represents, for example, a halogen atom, cyano group, alkyl group having 1 to 10 carbon atoms, alkenyl group having 2 to 10 carbon atoms, aryl group having 6 to 20 carbon atoms, optionally substituted amino group, -ORₐ group, -SR_{b} group or COOR_{c} group, and Rₐ, R_{b} and R_{c} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms.

Examples of the above-described halogen atom include a chlorine atom, bromine atom, fluorine atom, iodine atom and the like.

Among the above-described alkyl groups having 1 to 10 carbon atoms, examples of unsubstituted linear or branched alkyl groups having 1 to 10 carbon atoms include a methyl group, ethyl group, n-propyl group, isopropyl group, n-butyl group and the like.

Examples of the alkenyl group having 2 to 10 carbon atoms include a vinyl group, ethenyl group, 1-propenyl group, 2-propenyl group, 1-butenyl group, 2-butenyl group, 3-butenyl group and the like.

Examples of the aryl group having 6 to 20 carbon atoms include a phenyl group, naphthyl group and the like.

Examples of the optionally substituted amino group include an amino group, acetylamino group, benzoylamino group, methylamino group, dimethyl-amino group, benzylamino group, tert-butoxycarbonylamino group, benzyloxycarbonylamino group and the like.

Examples of Rₐ of the -ORₐ group include a hydrogen atom, alkylcarbonyl groups having 1 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group, and the like.

Examples of R_{b} of the -SR_{b} group include a hydrogen atom, alkylcarbonyl groups having 2 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group, and the like.

Examples of R_{c} of -COOR_{c} include alkyl groups having 1 to 10 carbon atoms such as a methyl group, ethyl group, n-propyl group, isopropyl group and the like, aralkyls having 7 to 20 carbon atoms such as a benzyl group, and the like.

Examples of specific structures of R include divalent groups of the following formulae, and the like.
-CH₂-, -(CH₂)₂-, -(CH₂)₃-, -(CH₂)₄-, (CH₃)₂C<, (Cl)₂C<, (F)₂C<, >CH(CO₂C₂H₅),

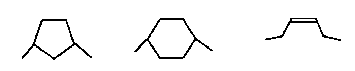

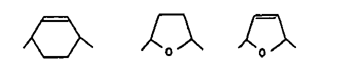

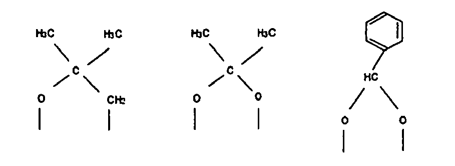

In the formula (2), R₃, R₄, R₅ and R₆ represent each independently a hydrogen atom, halogen atom, cyano group, optionally substituted alkyl group having 1 to 10 carbon atoms, optionally substituted alkenyl group having 2 to 10 carbon atoms, optionally substituted aryl group having 6 to 20 carbon atoms, optionally substituted amino group, -ORₐ group, -SR_{b} group or COOR_{c} group, and Rₐ, R_{b} and R_{c} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms.

Examples of the above-described halogen atom include a chlorine atom, bromine atom, fluorine atom, iodine atom and the like.

Among the above-described optionally substituted alkyl groups having 1 to 10 carbon atoms, examples of unsubstituted linear or branched alkyl groups having 1 to 10 carbon atoms include a methyl group, ethyl group, n-propyl group, isopropyl group, n-butyl group and the like.

Examples of substituted linear or branched alkyl groups having 1 to 10 carbon atoms include halogenated alkyl groups such as a chloromethyl group, dichloromethyl group, trichloromethyl group, fluoromethyl group, difluoromethyl group, trifluoromethyl group and the like; hydroxyalkyl groups such as a hydroxymethyl group or hydroxyethyl group and the like optionally substituted with a substituent such as an acetyl group, benzoyl group, methyl group, benzyl group, phenyl group, methoxymethyl group, trimethylsilyl group and the like; aminoalkyl groups such as an aminomethyl group, aminoethyl group and the like optionally substituted with a substituent such as an acetyl group, benzoyl group, methyl group, benzyl group, phenyl group, tert-butoxycarbonyl group, benzyloxycarbonyl group and the like; hydroxycarbonylalkyl groups such as a hydroxycarbonylmethyl group, hydroxycarbonylethyl group and the like optionally substituted with a substituent such as a methyl group, ethyl group, n-propyl group, i-propyl group, benzyl group and the like; aralkyl groups such as a phenylmethyl group, phenylethyl group and the like optionally substituted with a substituent such as a halogen atom, alkoxy group, -OH group, nitro group, cyano group, alkyl group having 1 t o 4 carbon atoms, aryl group and the like.

Further, examples of the optionally substituted alkenyl group having 2 to 10 carbon atoms include alkenyl groups having 2 to 7 carbon atoms such as a vinyl group, ethenyl group, 1-propenyl group, 2-propenyl group, 1-butenyl group, 2-butenyl group, 3-butenyl group and the like; hydroxycarbonylalkenyl groups such as a hydroxycarbonylethenyl group and the like optionally substituted with a substituent such as a methyl group, ethyl group, n-propyl group, isopropyl group, benzyl group and the like.

Examples of the optionally substituted aryl group having 6 to 20 carbon atoms include a phenyl group, naphthyl group and the like optionally substituted with a substituent such as a halogen atom, alkoxy group, hydroxyl group, nitro group, cyano group, alkyl group having 1 to 6 carbon atoms, aryl group having 6 to 20 carbon atoms and the like.

Examples of the optionally substituted amino group include amino groups optionally substituted with a substituent such as an acetyl group, benzoyl group, methyl group, benzyl group, tert-butoxycarbonyl group or benzyloxycarbonyl group and the like.

Examples of Rₐ of the -ORₐ group include a hydrogen atom, alkylcarbonyl groups having 1 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group, and the like.

Examples of R_{b} of the -SR_{b} group include a hydrogen atom, alkylcarbonyl groups having 2 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group, and the like.

Examples of R_{c} of -COOR_{c} include alkyl groups having 1 to 10 carbon atoms such as a methyl group, ethyl group, n-propyl group, isopropyl group and the like, aralkyl groups having 7 to 20 carbon atoms such as a benzyl group, and the like.

It is preferable that R₃ and R₄ represent a hydrogen atom.

It is preferable that R₅ and R₆ represent a hydrogen atom.

Examples of the above-described lactones(2) include 3-oxabicyclo[3.2.0]heptan-2-one, 3-oxabicyclo[3.3.0]octan-2-one, 8-oxabicyclo[4.3.0]nonan-7-one, 6,6-dimethyl-3-oxabicyclo[3.1.0]hexan-2-one, 6,6-dichloro-3-oxabicyclo[3.1.0]hexan-2-one, 6,6-difluoro-3-oxabicyclo[3.1.0]hexan-2-one, 6-ethoxycarbonyl-3-oxabicyclo[3.1.0]hexan-2-one, 1-phenyl-3-oxabicyclo[3.1.0]hexan-2-one, 4-oxatricyclo[5.2.1.0^{2,6}]deca-3-one, 4-oxatricyclo[5.2.2.0^{2,6}]undeca-3-one, 8-oxabicyclo[4.3.0]nonan-3-en-7-one, 4-oxatricyclo[5.2.1.0^{2,6}]deca-8-en-3-one, 4-oxatricyclo[5.2.2.0^{2,6}]undeca-8-en-3-one, 3,3-dimethyl-2,4,7-trioxabicyclo[3.3.0]octan-6-one, 3-phenyl-2,4,7-trioxabicyclo[3.3.0]octan-6-one, 3,3-dimethyl-2,7-dioxabicyclo[3.3.0]octan-6-one, 4,10-dioxatricyclo[5.2.1.0^{2,6}]deca-3-one, 4,10-dioxatricyclo[5.2.1.0^{2,6}]deca-8-en-3-one and the like, and optically active bodies of these compounds and the like.

The above-described lactones (2) may be, for example, synthesized by known methods, or synthesized by other methods.

In the step A, the carboxylic acids (4a), (4b) or (4c) can be obtained, for example, by reacting imides (3a), (3b) or (3c) and lactones(2).

This reaction can be carried out, if necessary, in a solvent, and further, if necessary, can be carried out by mixing with heating in the presence of a base in a solvent.

In the imides (3a), (3b) or (3c), A₁, A₂, A₃, A₄, A₅, A₆, A₇, A₈, A₉ and A₁₀ represent each independently a hydrogen atom, halogen atom, nitro group, alkyl group having 1 to 6 carbon atoms, alkenyl group having 2 to 4 carbon atoms, optionally substituted aryl group having 6 to 20 carbon atoms, optionally substituted acyl group having 2 to 4 carbon atoms, or -ORₐ group. Here, Rₐ represents the same meaning as described above. In the imides (3b), A₅, A₆, A₇ and A₈ may be connected to form a cyclic structure, and likewise in the imides (3c), A₉ and A₁₀ may be connected to form a cyclic structure.

Examples of the halogen atom include a chlorine atom, bromine atom, fluorine atom, iodine atom and the like.

Examples of the alkyl group having 1 to 6 carbon atoms include a methyl group, ethyl group, n-propyl group, isopropyl group, n-butyl group, cyclohexyl group and the like.

Examples of the alkenyl group having 2 to 4 carbon atoms include a vinyl group, ethenyl group, 1-propenyl group, 2-propenyl group, 1-butenyl group, 2-butenyl group, 3-butenyl group and the like.

Examples of the optionally substituted aryl group having 6 to 20 carbon atoms include a phenyl group, naphthyl group and the like optionally substituted with a substituent such as a halogen atom, alkoxy group, hydroxyl group, nitro group, cyano group, alkyl group having 1 to 6 carbon atoms, aryl group having 6 to 14 carbon atoms and the like.

Examples of the optionally substituted acyl group having 2 to 4 carbon atoms include an acetyl group, chloroacetyl group, pivaloyl group, benzyl group and the like.

Examples of the Rₐ of the -ORₐ group include a hydrogen atom, alkylcarbonyl groups having 2 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group, and the like.

In the imides (3a), (3b) or (3c), M represents an alkali metal atom such as sodium, potassium and the like, or a hydrogen atom.

Examples of the above-described imides (3a), (3b) or (3c) include phthalimide, 4,5-dichlorophthalimide, 4-nitrophthalimide, succinic imide, 3-acetyloxy-2,5-pyrrolidinedione, 3,3,4,4-tetramethyl-2,5-pyrrolidinedione, 4-cyclohexene-1,2-dicarboxylic imide, hexahydrophthalimide, 3,4,5,6-tetrahydrophthalimide, maleic imide, 2,3-dimethylmaleic imide, and sodium salts and potassium salts of these imides, and the like.

Of the imides (3a), (3b) or (3c), the imides (3a) are preferably used, phthalimide, phthalimide sodium and phthalimide potassium are more preferably used, and phthalimide potassium is particularly preferably used.

The use amount of the above-described imides (3a), (3b) or (3c) is usually in the range of 0.8 to 5 mole ratio, preferably 1 to 2 mole ratio with respect to the lactones (2).

In the above-described step A, an amide solvent of the following formula (6): 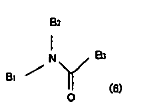 (wherein, B₁ and B₂ represent each independently a hydrogen atom or alkyl group having 1 to 5 carbon atoms, B₃ represents a hydrogen atom, alkyl group having 1 to 10 carbon atoms or aryl group having 6 to 20 carbon atoms, and B₂ and B₃ may be connected to form a cyclic structure.) is preferably used as the reaction solvent.

It is more preferable that B₃ is an alkyl group having 1 to 10 carbon atoms or aryl group having 6 to 20 carbon atoms.

Examples of the above-described amide solvent (6) include N,N-dimethylformamide, N-methylacetamide, N,N-dimethylacetamide, N,N-dimethylpropionamide, N,N-dimethylbutylamide, N,N-dimethylacrylamide, 1-methyl-2-pyrrolidinone, 1-methyl-2-piperidone, N-methylcaprolactam, 1-methyl-2-pyridone and the like. The amide solvent (6) includes, particularly preferably, N,N-dimethylacetamide.

The use amount of the above-described amide solvent (6) is usually in the range of 0.5 to 50 weight ratio, preferably 1 to 10 weight ratio with respect to the lactones (2).

In addition to the above-described amide solvent (6), a solvent inactive to the reaction may also be used together.

The reaction in the step A is carried out by mixing any of imides (3a), imides (3b) and imides (3c), lactones(2), and if necessary, an amide solvent(6), then, if necessary, heating the mixture. The order of charging of the above-described raw material compounds and solvent is not particularly restricted, and there are mentioned, for example, a method in which any of the above-described imides and an amide solvent(6) are mixed, and lactones(2) are dropped into this mixture while heating, and other methods.

When M in the imides (3a), (3b) or (3c) is a hydrogen atom, a base may be added to these imides and reacted.

Examples of the base in this case include alkali metal carbonates such as sodium carbonate, potassium carbonate, cesium carbonate and the like, alkali metal bicarbonates such as sodium hydrogen carbonate, potassium hydrogen carbonate and the like, alkali metal hydrides such as sodium hydride, potassium hydride and the like, alkali metals such as metal sodium, metal potassium, and the like. The use amount of the above-described base is usually in the range of 0.5 to 10 mole ratio, preferably 1 to 5 mole ratio with respect to the above-described imides.

In the reaction of the step A, for example, the above-described base and the above-described imides may be mixed in an amide solvent (6) before adding lactones (2), or a mixture of the above-described imides and lactones (2) may be added into a solution composed of a base and an amide solvent (6).

The reaction temperature in the step A is usually in the range of 50 to 300°C, preferably 80 to 200°C.

Thus, the correspondent carboxylic acids (4a), (4b) or (4c) are produced.

After completion of the reaction in the step A, the above-described carboxylic acids in which M is an alkali metal may be used as they are in the step B, or the reaction liquid obtained in the step A may be once subjected to a crystallization operation, to isolate the reaction product.

After completion of the reaction in the step A, a post-treatment operation may be carried out for removing inorganic materials and the like. Examples of the above-described post-treatment operation include an operation for filtrating unnecessary materials such as inorganic salts and the like, an operation in which water and an organic solvent separable from water are added to the reaction liquid, the mixture is neutralized using an acid, then, an aqueous layer is removed, for the purpose of obtaining free carboxylic acids of the above-described carboxylic acids, and other operations. From an organic layer obtained in the operation for removing an aqueous layer, the above-described free carboxylic acids may be once isolated by distilling off a solvent contained in this organic layer.

The above-described carboxylic acids (4a), (4b) or (4c) may be, while maintaining the solution state, used in the subsequently step B. They may also be purified by a method such as column chromatography, re-crystallization and the like.

Examples of the above-described organic solvent separable from water include aliphatic hydrocarbon solvents (hexane, heptane, cyclohexane and the like), aromatic solvents (toluene, xylene, monochlorobenzene, dichlorobenzene and the like), ether solvents (methyl tert-butyl ether, 1,2-dimethoxyethane and the like), halogenated hydrocarbon solvents (dichloromethane, dichloroethane, chlorobutane and the like), ketone solvents (methyl ethyl ketone, methyl isobutyl ketone and the like), ester solvents (methyl acetate, ethyl acetate, butyl acetate and the like), and so forth. These solvents may be used in admixture of two or more.

The use amount of the organic solvent separable from water is usually in the range of 1 to 100 weight ratio, preferably 2 to 20 weight ratio with respect to lactones (2).

Examples of the acid to be used in the post-treatment operation include inorganic acids (hydrogen chloride, hydrogen bromide, sulfuric acid, phosphoric acid and the like) and organic acids (acetic acid, citric acid, methanesulfonic acid and the like). The use amount of these acids is, when M in the imides used in the reaction is an alkali metal atom, usually in the range of 0.5 to 3 mole ratio, preferably 1 to 2 mole ratio with respect to this alkali metal atom. When M in the imides used in the reaction is a hydrogen atom, it is usually in the range of 0.5 to 3 mole ratio, preferably 1 to 2 mole ratio with respect to the base used.

The post-treatment is carried out at a temperature at which a phthaloyl group in the above-described imides does not undergo hydrolysis. The temperature in the post-treatment is usually in the range of 0 to 100°C, preferably 10 to 80°C when water coexists.

R, R₃, R₄, R₅, R₆, A₁, A₂, A₃, A₄, A₅, A₆, A₇, A₈, A₉, A₁₀ and M in the carboxylic acids (4a), (4b) or (4c) represent the same meanings as for R, R₃, R₄, R₅ and R₆ defined in the lactones(2) and A₁, A₂, A₃, A₄, A₅, A₆, A₇, A₈, A₉, A₁₀ and M defined in the imides (3a), (3b) or (3c).

Examples of the above-described carboxylic acids (4a), (4b) or (4c) include 2-[(1,3-dihydro-1,3-dioxo-2H-isoindol-2-yl)methyl]cyclobutanecarboxylic acid, 2-[(1,3-dihydro-1,3-dioxo-2H-isoindol-2-yl)methyl]cyclopentanecarboxylic acid, 2-[(1,3-dihydro-1,3-dioxo-2H-isoindol-2-yl)methyl]cyclohexanecarboxylic acid, 2,2-dimethyl-3-[(1,3-dihydro-1,3-dioxo-2H-isoindol-2-yl)methyl]cyclopropanecarboxylic acid, 2,2-dichloro-3-[(1,3-dihydro-1,3-dioxo-2H-isoindol-2-yl)methyl]cyc1opropanecarboxylic acid, 2,2-difluoro-3-[(1,3-dihydro-1,3-dioxo-2H-isoindol-2-yl)methyl]cyclopropanecarboxylic acid, 2-ethoxycarbonyl-3-[(1,3-dihydro-1,3-dioxo-2H-isoindol-2-yl)methyl]cyclopropanecarboxylic acid, 2-[(1,3-dihydro-1,3-dioxo-2H-isoindol-2-yl)methyl]-1-phenylcyclopropanecarboxylic acid, 3-[(1,3-dihydro-1,3-dioxo-2H-isoindol-2-yl)methyll-bicyclo[2.2.1]heptane-2-carboxylic acid, 3-[(1,3-dihydro-1,3-dioxo-2H-isoindol-2-yl)methyl]-bicyclo[2.2.2]octane-2-carboxylic acid, 6-[(1,3-dihydro-1,3-dioxo-2H-isoindol-2-yl)methyl]-cyclohex-3-enecarboxylic acid, 3-[(1,3-dihydro-1,3-dioxo-2H-isoindol-2-yl)methyl]-bicyclo[2.2.1]hept-5-ene-2-carboxylic acid, 3-[(1,3-dihydro-1,3-dioxo-2H-isoindol-2-yl)methyl]-bicyclo[2.2.2]oct-5-ene-2-carboxylic acid, 5-[(1,3-dihydro-1,3-dioxo-2H-isoindol-2-yl)methyl]-2,2-dimethyl-1,3-dioxolan-4-carboxylic acid, 5-[(1,3-dihydro-1,3-dioxo-2H-isoindol-2-yl)methyl]-2-phenyl-1,3-dioxolan-4-carboxylic acid, 2-[(1,3-dihydro-1,3-dioxo-2H-isoindol-2-yl)methyl]-5,5-dimethyl-tetrahydrofuran-3-carboxylic acid, 3-[(1,3-dihydro-1,3-dioxo-2H-isoindol-2-yl)methyl]-7-oxabicyclo[2.2.1]heptane-2-carboxylic acid, 3-[(1,3-dihydro-1,3-dioxo-2H-isoindol-2-yl)methyl]-7-oxabicyclo[2.2.1]hept-5-ene-2-carboxylic acid, 2,2-dimethyl-3-[(5,6-dichloro-1,3-dihydro-1,3-dioxo-2H-isoindol-2-yl)methyl]cyclopropanecarboxylic acid, 2,2-dimethyl-3-[(1,3-dihydro-5-nitro-1,3-dioxo-2H-isoindol-2-yl)methyl]cyclopropanecarboxylic acid, 2,2-dimethyl-3-[(2,5-dioxo-1-pyrrolidinyl)methyl]cyclopropanecarboxylic acid, 2,2-dimethyl-3-[(3-acetyloxy-2,5-dioxo-1-pyrrolidinyl)methyl]cyclopropanecarboxylic acid, 2,2-dimethyl-3-[(3,3,4,4-tetramethyl-2,5-dioxo-1-pyrrolidinyl)methyl]cyclopropanecarboxylic acid, 2,2-dimethyl-3-[(3a,4,7,7a-tetrahydro-1,3-dioxo-2H-isoindol-2-yl)methyl]cyclopropanecarboxylic acid, 2,2-dimethyl-3-[(hexahydro-1,3-dioxo-2H-isoindol-2-yl)methyl]cyclopropanecarboxylic acid, 2,2-dimethyl-3-[(4,5,6,7-tetrahydro-1,3-dioxo-2H-isoindol-2-yl)methyl]cyclopropanecarboxylic acid, 2,2-dimethyl-3-[(1,3-dioxo-2H-pyrrol-2-yl)methyl]cyclopropanecarboxylic acid, 2,2-dimethyl-3-[(4,5-dimethyl-1,3-dioxo-2H-pyrrol-2-yl)methyl]cyclopropanecarboxylic acid, salts of alkali metals such as sodium, potassium and the like of the above-exemplified compound, and optically active bodies thereof and the like.

The step B is a step of esterifying the carboxylic acids (4a), (4b) or (4c) obtained in the step A to obtain the correspondent esters of the formula (5a), (5b) or (5c) [hereinafter, abbreviated as esters (5a), (5b) or (5c), respectively, in some cases].

In the step B, a general esterifying method can be used, and examples of the esterifying agent include alcohols of the following formula (7)

R₇-OH (7)

(wherein, R₇ represents an alkyl group having 1 to 10 carbon atoms, alkenyl group having 2 to 10 carbon atoms or aralkyl group having 7 to 20 carbon atoms.) [hereinafter, abbreviated as alcohols (7) in some cases],
and compounds obtained by substitution of a hydroxyl group of alcohols (7) with a halogen atom, and the like. The compounds obtained by substitution of a hydroxyl group of alcohols (7) with a halogen atom include halogenated alkyls having 1 to 10 carbon atoms, halogenated alkenyls having 2 to 10 carbon atoms, halogenated aralkyls having 7 to 20 carbon atoms and the like.

Examples of R₇ in the alcohols (7) include alkyl groups having 1 to 10 carbon atoms such as a methyl group, ethyl group, n-propyl group, isopropyl group, n-butyl group, isobutyl group, sec-butyl group, tert-butyl group and the like, alkenyl groups having 2 to 10 carbon atoms such as an allyl group, 3-butenyl group and the like, aralkyl groups having 7 to 20 carbon atoms such as a benzyl group, and the like. The aralkyl group having 7 to 20 carbon atoms optionally has a substituent.

Examples of the above-described alcohols (7) include methanol, ethanol, 1-propanol, 2-propanol, 1-butanol, 2-butanol, 2-methyl-1-propanol, 2-methyl-2-propanol, allyl alcohol, benzyl alcohol, p-methoxybenzyl alcohol and the like.

The above-described alcohols (7) can be used concurrently as the reaction solvent. The use amount of the alcohols (7) is usually in the range of 1 to 50 weight ratio, preferably 2 to 10 weight ratio with respect to carboxylic acids (4a), (4b) or (4c).

In the step B, if necessary, there can be used organic solvents such as aliphatic hydrocarbon solvents (hexane, heptane, cyclohexane and the like), aromatic hydrocarbon solvents (toluene, xylene, monochlorobenzene, dichlorobenzene and the like), ether solvents (tetrahydrofuran, methyl tert-butyl ether, 1,4-dioxane, 1,2-dimethoxyethane and the like), halogenated hydrocarbon solvents (dichloromethane, dichloroethane, chlorobutane and the like), or the amide solvent (6) used in the step A, and the like. This organic solvent may be a mixture of two or more compounds.

Examples of the method of esterifying carboxylic acids (4a), (4b) or (4c) using alcohols (7) include a dehydrating condensation method using an acid catalyst, an acid chloride method using a chlorinating agent, a dehydrating condensation method using a dehydrating condensation agent, an acid anhydride method using an alkyl chlorocarbonate, and the like.

Examples of the acid catalyst to be used in the above-described dehydrating condensation method include inorganic acids such as hydrogen chloride, hydrogen bromide, sulfuric acid, phosphoric acid and the like, organic acids such as p-toluenesulfonic acid, camphor-10-sulfonic acid and the like, acidic ion exchange resins such as Amberlite, Amberlyst, and the like.

The amount of the acid catalyst to be used in the above-described dehydrating condensation method is usually in the range of 0.01 to 1 weight ratio with respect to carboxylic acids (4a), (4b) or (4c).

Examples of the chlorinating agent to be used in the above-described acid chloride method include thionyl chloride, phosphorus trichloride, phosphorus pentachloride, oxalyl chloride and the like.

The dehydrating condensation agent to be used in the above-described dehydrating condensation method includes dicyclohexylcarbodiimide, 1-ethyl-3-(3-dimethylaminopyrrolyl)-carbodiimide and the like.

The alkyl chlorocarbonate to be used in the above-described acid anhydride method includes ethyl chlorocarbonate, isopropyl chlorocarbonate and the like.

The use amount of the chlorinating agent to be used in the acid chloride method, the dehydrating condensation agent to be used in the dehydrating condensation method and the alkyl chlorocarbonate to be used in the acid anhydride method is usually in the range of 0.8 to 10 mole ratio, preferably 1 to 5 mole ratio with respect to carboxylic acids (4a), (4b) or (4c).

When the esterifying reaction of carboxylic acids (4a), (4b) or (4c) is carried out using a halogenated alkyl obtained by substituting a hydroxyl group of alcohols (7) with a halogen atom, or correspondent halogenated alkenyl or halogenated aralkyl, it is preferable that the reaction is carried out in the presence of an organic solvent and a base. When the carboxylic acids (4a), (4b) or (4c) obtained in the step A are an alkali metal salt, the base described above is unnecessary in some cases.

Examples of the above-described halogenated alkyl and correspondent halogenated alkenyl or halogenated aralkyl include bromoethane, 1-bromopropane, 2-bromopropane, 1-bromobutane, 2-bromobutane, 1-bromo-2-methylpropane, 2-bromo-2-methylpropane, allyl bromide, benzyl bromide and p-methoxybenzyl bromide, compounds obtained by substitution of a bromine atom in the above-exemplified compounds with a chlorine atom or iodine atom, and the like.

When a base is used in the esterifying reaction of the step B, examples of the base to be used include alkali metal hydroxides (sodium hydroxide, potassium hydroxide and the like), alkali metal carbonates (sodium carbonate, potassium carbonate, cesium carbonate and the like), alkali metal bicarbonates (sodium hydrogen carbonate, potassium hydrogen carbonate and the like), alkali metal alcoholates (sodium methoxide, sodium ethoxide and the like), alkali metal hydrides (sodium hydride, potassium hydride and the like), organic bases (triethylamine, diisopropylethylamine, 1,8-diazabicyclo[5.4.0]undeca-7-ene, pyridine and the like). The use amount of the base is usually in the range of 0 to 10 mole ratio, preferably 1 to 5 mole ratio with respect to carboxylic acids (4a), (4b) or (4c).

Examples of the organic solvent to be used in the esterifying reaction include aliphatic hydrocarbon solvents (hexane, heptane, cyclohexane and the like), aromatic solvents (toluene, xylene, monochlorobenzene, dichlorobenzene and the like), ether solvents (tetrahydrofuran, methyl tert-butyl ether, 1,4-dioxane, 1,2-dimethoxyethane and the like), halogenated hydrocarbon solvents (dichloromethane, dichloroethane, chlorobutane and the like), ketone solvents (acetone, methyl ethyl ketone, methyl isobutyl ketone and the like), nitrile solvents (acetonitrile, propionitrile and the like), amide solvents (N,N'-dimethylformamide, N,N'-dimethylacetamide, N-methylpyrrolidinone and the like). The use amount of the organic solvent is usually in the range of 1 to 100 weight ratio, preferably 2 to 20 weight ratio with respect to carboxylic acids (4a), (4b) or (4c).

In the above-described esterifying reaction, an acid chloride method using the above-described alcohols (7) and a chlorinating agent is preferably used.

For example, into a solution composed of any of carboxylic acids (4a), (4b) and (4c) obtained in the step A and the above-described alcohols (7), a chlorinating agent is dropped, thereby obtaining any of esters (5a), (5b) and (5c).

The esterifying reaction may also be carried out by, distilling off a solvent if necessary from a solution containing any of carboxylic acids (4a), (4b) or (4c) obtained in the step A, adding alcohols (7), then, dropping a chlorinating agent. The above-described esterifying reaction may also be carried out by adding a chlorinating agent to a solution composed of any of carboxylic acids (4a), (4b) or (4c) and an organic solvent, then, dropping alcohols (7).

The temperature of the esterifying reaction is usually in the range from -20 to 100°C, preferably from 0°C to temperature not higher than the boiling point of alcohols (7) or reaction solvent.

Thus, esters (5a), (5b) or (5c) are obtained. When esters (5a), (5b) or (5c) deposit as a crystal after the above-described esterifying reaction, the crystal may be isolated by filtering the slurry. Further, the deposition rate of the crystal may be enhanced by distilling off the reaction solvent and the like or dropping a poor solvent, before performing filtration.

Even if the crystal of esters (5a), (5b) or (5c) does not deposit in the reaction liquid, a crystal can be deposited by the above-described distilling off of a solvent and the like or dropping of a poor solvent, and filtrated, thereby performing isolation thereof.

Further, in the above-described esterifying reaction, surplus chlorinating agent and by-produced hydrogen chloride and the like can also be removed from the resultant solution, by performing a washing operation using water and an organic solvent separable from water or using a basic aqueous solution. For example, there are mentioned a method in which a solvent separable from water is added to a solution obtained after the above-described esterifying reaction, water or basic aqueous solution is mixed, then, liquid partitioning is carried out, and other methods. It may also be permissible that before adding a solvent separable from water, the solvent used in the reaction may be previously distilled off. It may also be permissible that the resultant solution is dropped into water or basic aqueous solution, then, a solvent separable from water is added to cause liquid partitioning. The washing operation with water or basic aqueous solution may be repeated.

Examples of the organic solvent separable from water include aliphatic hydrocarbon solvents (hexane, heptane, cyclohexane and the like), aromatic solvents (toluene, xylene, monochlorobenzene, dichlorobenzene and the like), ether solvents (methyl tert-butyl ether, 1,2-dimethoxyethane and the like), halogenated hydrocarbon solvents (dichloromethane, dichloroethane, chlorobutane and the like), ketone solvents (methyl ethyl ketone, methyl isobutyl ketone and the like), ester solvents (methyl acetate, ethyl acetate, butyl acetate and the like).

The use amount of the organic solvent is usually in the range of 1 to 100 weight ratio, preferably 2 to 20 weight ratio with respect to carboxylic acids (4a), (4b) or (4c).

Examples of the base to be used as the basic aqueous solution include alkali metal hydroxides (sodium hydroxide, potassium hydroxide and the like), alkali metal carbonates (sodium carbonate, potassium carbonate and the like), alkali metal bicarbonates (sodium hydrogen carbonate, potassium hydrogen carbonate and the like), alkali metal phosphates (trisodium phosphate, disodium hydrogen phosphate, tripotassium phosphate, dipotassium hydrogen phosphate and the like), organic bases (pyridine, triethylamine and the like) and ammonia, and the like. The use amount of the above-described base is not particularly restricted, and usually in the range of 0.5 to 20 mole ratio, preferably 1 to 10 mole ratio with respect to the chlorinating agent used.

The above-described operations of washing and liquid partitioning are preferably carried out in a pH range in which an ester group and a phthaloyl group of esters (5a), (5b) or (5c) are not hydrolyzed. The no-hydrolysis pH range is usually a pH range from 1 to 10. For performing the above-described operations of washing and liquid partitioning in this pH rang, there are mentioned, for example, a method in which a solution obtained in the esterifying reaction is dropped into a basic aqueous solution having pH regulated in the above-described range, a method in which a reaction solution and a basic aqueous solution are simultaneously poured into water while controlling pH in the above-described range.

An organic layer containing the resultant esters (5a), (5b) or (5c) may be used as it is in the step C, or a solvent thereof may be concentrated before effecting the step C. It may be further purified by column chromatography, re-crystallization and the like.

R, R₃, R₄, R₅, R₆, R₇, A₁, A₂, A₃, A₄, A₅, A₆, A₇, A₈, A₉ and A₁₀ in the esters (5a), (5b) or (5c) represent the same meanings as for R, R₃, R₄, R₅ and R₆ defined in the lactones (2), A₁, A₂, A₃, A₄, A₅, A₆, A₇, A₈, A₉, A₁₀ defined in the imides (3a), (3b) or (3c), and R₇ defined in the alcohols (7).

Examples of the esters (5) include methyl 2-[(1,3-dihydro-1,3-dioxo-2H-isoindol-2-yl)methyl]cyclobutanecarboxylate, methyl 2-[(1,3-dihydro-1,3-dioxo-2H-isoindol-2-yl)methyl]cyclopentanecarboxylate, methyl 2-[(1,3-dihydro-1,3-dioxo-2H-isoindol-2-yl)methyl]cyclohexanecarboxylate, methyl 2,2-dimethyl-3-[(1,3-dihydro-1,3-dioxo-2H-isoindol-2-yl)methyl]cyclopropanecarboxylate, methyl 2,2-dichloro-3-[(1,3-dihydro-1,3-dioxo-2H-isoindol-2-yl)methyl]cyclopropanecarboxylate, methyl 2,2-difluoro-3-[(1,3-dihydro-1,3-dioxo-2H-isoindol-2-yl)methyl]cyclopropanecarboxylate, methyl 2-ethoxycarbonyl-3-[(1,3-dihydro-1,3-dioxo-2H-isoindol-2-yl)methyl]cyclopropanecarboxylate, methyl 2-[(1,3-dihydro-1,3-dioxo-2H-isoindol-2-yl)methyl]-1-phenylcyclopropanecarboxylate, methyl 3-[(1,3-dihydro-1,3-dioxo-2H-isoindol-2-yl)methyl]-bicyclo[2.2.1]heptane-2-carboxylate, methyl 3-[(1,3-dihydro-1,3-dioxo-2H-isoindol-2-yl)methyl]-bicyclo[2.2.2]octane-2-carboxylate, methyl 6-[(1,3-dihydro-1,3-dioxo-2H-isoindol-2-yl)methyl]-cyclohex-3-enecarboxylate, methyl 3-[(1,3-dihydro-1,3-dioxo-2H-isoindol-2-yl)methyl]-bicyclo[2.2.1]hept-5-ene-2-carboxylate, methyl 3-[(1,3-dihydro-1,3-dioxo-2H-isoindol-2-yl)methyl]-bicyclo[2.2.2]oct-5-ene-2-carboxylate, methyl 5-[(1,3-dihydro-1,3-dioxo-2H-isoindol-2-yl)methyl]-2,2-dimethyl-1,3-dioxolan-4-carboxylate, methyl 5-[(1,3-dihydro-1,3-dioxo-2H-isoindol-2-yl)methyl]-2-phenyl-1,3-dioxolan-4-carboxylate, methyl 2-[(1,3-dihydro-1,3-dioxo-2H-isoindol-2-yl)methyl]-5,5-dimethyltetrahydrofuran-3-carboxylate, methyl 3-[(1,3-dihydro-1,3-dioxo-2H-isoindol-2-yl)methyl]-7-oxabicyclo[2.2.1]heptane-2-carboxylate, methyl 3-[(1,3-dihydro-1,3-dioxo-2H-isoindol-2-yl)methyl]-7-oxabicyclo[2.2.1]hept-5-ene-2-carboxylate, methyl 2,2-dimethyl-3-[(5,6-dichloro-1,3-dihydro-1,3-dioxo-2H-isoindol-2-yl)methyl]cyclopropanecarboxylate, methyl 2,2-dimethyl-3-[(1,3-dihydro-5-nitro-1,3-dioxo-2H-isoindol-2-yl)methyl]cyclopropanecarboxylate, methyl 2,2-dimethyl-3-[(2,5-dioxo-1-pyrrolidinyl)methyl]cyclopropanecarboxylate, methyl 2,2-dimethyl-3-[(3-acetyloxy-2,5-dioxo-1-pyrrolidinyl)methyl]cyclopropanecarboxylate, methyl 2,2-dimethyl-3-[(3,3,4,4-tetramethyl-2,5-dioxo-1-pyrrolidinyl)methyl]cyclopropanecarboxylate, methyl 2,2-dimethyl-3-[(3a,4,7,7a-tetrahydro-1,3-dioxo-2H-isoindol-2-yl)methyl]cyclopropanecarboxylate, methyl 2,2-dimethyl-3-[(hexahydro-1,3-dioxo-2H-isoindol-2-yl)methyl]cyclopropanecarboxylate, methyl 2,2-dimethyl-3-[(4,5,6,7-tetrahydro-1,3-dioxo-2H-isoindol-2-yl)methyl]cyclopropanecarboxylate, methyl 2,2-dimethyl-3-[(1,3-dioxo-2H-pyrrol-2-yl)methyl]cyclopropanecarboxylate, methyl-2,2-dimethyl-3-[(4,5-dimethyl-1,3-dioxo-2H-pyrrol-2-yl)methyl]cyclopropanecarboxylate, and the like, and compounds obtained by converting a methyl ester group of the above-exemplified compounds into an ethyl ester, n-propyl ester, isopropyl ester, n-butyl ester, isobutyl ester, sec-butyl ester, tert-butyl ester, allyl ester, 3-butenyl ester, benzyl ester or p-methoxybenzyl ester and the like, and optically active bodies of these compounds.

The step C is a step of reacting the esters (5a), (5b) or (5c) obtained in the step B and amines to obtain lactams of the formula (1) [hereinafter, abbreviated as lactams(1) in some cases].

As the de-protecting method of N-protective group, there is mentioned, for example, a method of reacting amines of the following formula (8):

R₈-NH₂ (8)

(wherein, R₈ represents an optionally substituted amino group, or an alkyl group having 1 to 5 carbon atoms optionally substituted with an amino group) [hereinafter, abbreviated as amines (8) in some cases] and esters (5a), (5b) or (5c).

Examples of R₈ in the amines (8) include optionally substituted amino groups (amino group, methylamino group, ethylamino group, phenylamino group and the like), unsubstituted alkyl groups having 1 to 5 carbon atoms (methyl group, ethyl group, n-propyl group, isopropyl group, n-butyl group, isobutyl group, sec-butyl group, tert-butyl group and the like), alkyl groups having 1 to 5 carbon atoms substituted with an amino group (2-aminoethyl group, N,N-dimethyl-3-aminopropyl group and the like), and so forth.

Example of the above-described amines (8) include hydrazines (hydrazine, methylhydrazine, ethylhydrazine, phenylhydrazine and the like) and hydrates thereof, alkylamines (methylamine, ethylamine, n-propylamine, isopropylamine, n-butylamine, isobutylamine, sec-butylamine, tert-butylamine, 1,2-ethylenediamine, N,N-dimethyl-1,3-propanediamine and the like), and so forth.

The use amount of the above-described amines (8) is usually in the range of 1 to 10 mole ratio, preferably 1 to 5 mole ratio with respect to esters (5a), (5b) or (5c).

The lactamization reaction in the step C is usually carried out in the presence of a solvent. As the solvent, water, an organic solvent, or a mixed solvent of water and organic solvent is used.

Examples of the above-described organic solvent include alcohol solvents (methanol, ethanol, 2-propanol, ethylene glycol and the like), amide solvents (N,N'-dimethylformamide, N,N'-dimethylacetamide, N-methylpyrrolidinone and the like), ether solvents (tetrahydrofuran, methyl tert-butyl ether, 1,4-dioxane, 1,2-dimethoxyethane and the like), amine solvents (triethylamine, tri-n-butylamine, pyridine and the like), nitrile solvents (acetonitrile, propionitrile and the like), halogenated hydrocarbon solvents (dichloromethane, dichloroethane, chlorobutane and the like), aliphatic hydrocarbon solvents (hexane, heptane, cyclohexane and the like), aromatic solvents (toluene, xylene, monochlorobenzene, dichlorobenzene and the like), ester solvents (methyl acetate, ethyl acetate, butyl acetate and the like), and so forth. These organic solvents may be used in admixture of two or more. Of the above-described solvents, organic solvents miscible with water or mixtures of such organic solvents with water are preferably used. Examples of the solvents miscible with water include alcohol solvents, amide solvents, ether solvents, amine solvents, nitrile solvents, and the like.

The use amount of the above-described solvents is usually in the range of 1 to 100 weight ratio, preferably 2 to 20 weight ratio with respect to esters (5a), (5b) or (5c).

Examples of the lactamization reaction include a method in which esters (5a), (5b) or (5c), amines (8) and a reaction solvent are mixed and the mixture is adjusted to desired temperature, a method in which amines (8) are singly or a solution composed of amines (8) and a reaction solvent is dropped into a solution composed of esters (5a), (5b) or (5c) and a solvent, and other methods. The reaction temperature in the lactamization is usually from 0°C to temperature not higher than the boiling point of the solvent, preferably in the range of 10 to 100°C.

By the above-described lactamization reaction, lactams (1) are obtained. After completion of the reaction, there may be carried out an operation for removing surplus amount of amines (8) or by-products generated by de-protection of an N-protective group of esters (5a), (5b) or (5c).

For removing surplus amount of amines (8) out of the system, distillation may be carried out, or water-washing may be carried out after substituting the reaction solvent by a solvent separable from water. If the latter case is carried out in the presence of an acid, efficiency of removal of amines (8) can be enhanced. It may also be permissible that after completion of the lactamization reaction, an acid which will form a salt with amines (8) is added, and if necessary, the solvent is distilled off, then, a salt produced from the amines (8) and the acid is removed. Removal of the salt in this case is carried out by filtration.

Examples of the solvent separable from water include aliphatic hydrocarbon solvents (hexane, heptane, cyclohexane and the like), aromatic solvents (toluene, xylene, monochlorobenzene, dichlorobenzene and the like), ether solvents (methyl tert-butyl ether, 1,2-dimethoxyethane and the like), halogenated hydrocarbon solvents (dichloromethane, dichloroethane, chlorobutane and the like), ketone solvents (methyl ethyl ketone, methyl isobutyl ketone and the like), ester solvents (methyl acetate, ethyl acetate, butyl acetate and the like), and so forth.

The use amount of these solvents is usually in the range of 1 to 100 weight ratio, preferably 2 to 20 weight ratio with respect to esters (5a), (5b) or (5c).

Examples of the acid to form a salt with amines (8) in removing the amines (8) by washing with water include inorganic acids (hydrogen chloride, hydrogen bromide, sulfuric acid, phosphoric acid and the like) and organic acids (acetic acid, citric acid, methanesulfonic acid, p-toluenesulfonic acid and the like). The use amount of these acids is usually in the range of 0.5 to 20 mole ratio, preferably 1 to 5 mole ratio with respect to surplus amount of amines (8).

By-products generated in de-protecting an N-protective group in esters (5a), (5b) or (5c) may be, in the case for example of deposition of the above-described by-products after completion of the lactamization reaction, advantageously removed as they are by filtration. It may also be permissible that the reaction solvent is distilled off to increase the deposition amount of the by-products, then, the by-products are removed by filtration. Alternatively, it may also be permissible to perform substitution by a solvent which is capable of dissolving lactams (1) and depositing the by-products, before removal by filtration. Examples of the solvent which is capable of dissolving lactams (1) and depositing the by-products include aliphatic hydrocarbon solvents (hexane, heptane, cyclohexane and the like), aromatic solvents (toluene, xylene, monochlorobenzene, dichlorobenzene and the like), ether solvents (methyl tert-butyl ether, 1,2-dimethoxyethane and the like), halogenated hydrocarbon solvents (dichloromethane, dichloroethane, chlorobutane and the like), ketone solvents (methyl ethyl ketone, methyl isobutyl ketone and the like), ester solvents (methyl acetate, ethyl acetate, butyl acetate and the like), and so forth.

The use amount of the solvent which is capable of dissolving lactams (1) and depositing the by-products is usually in the range of 1 to 100 weight ratio, preferably 2 to 20 weight ratio with respect to esters (5a), (5b) or (5c).

Thus, a solution containing lactams (1) is obtained.

For isolation of lactams (1), for example, a method of concentrating a solvent in the above-described solution, and other methods are mentioned. Lactams (1) after concentration may be purified by column chromatography, re-crystallization and the like.

R, R₃, R₄, R₅ and R₆ in the lactams (1) represent the same meanings as for R, R₃, R₄, R₅ and R₆ defined in the lactones (2).

Examples of the lactams (1) include 3-azabicyclo[3.2.0]heptan-2-one, 3-azabicyclo[3.3.0]octan-2-one, 8-azabicyclo[4.3.0]nonan-7-one, 6,6-dimethyl-3-azabicyclo[3.1.0]hexan-2-one, 6,6-dichloro-3-azabicyclo[3.1.0]hexan-2-one, 6,6-difluoro-3-azabicyclo[3.1.0]hexan-2-one, 6-ethoxycarbonyl-3-azabicyclo[3.1.0]hexan-2-one, 1-phenyl-3-azabicyclo[3.1.0]hexan-2-one, 4-azatricyclo[5.2.1.0^{2,6}]deca-3-one, 4-azatricyclo[5.2.2.0^{2,6}]undeca-3-one, 8-azabicyclo[4.3.0]nonan-3-en-7-one, 4-azatricyclo[5.2.1.^{2,6}]deca-8-en-3-one, 4-azatricyclo[5.2.2.0^{2,6}]undeca-8-en-3-one, 3,3-dimethyl-2,4-dioxa-7-azabicyclo[3.3.0]octan-6-one, 3-phenyl-2,4-dioxa-7-azabicyclo[3.3.0]octan-6-one, 3,3-dimethyl-2-oxa-7-azabicyclo[3.3.0]octan-6-one, 4-aza-10-oxatricyclo[5.2.1.0^{2,6}]deca-3-one, 4-aza-10-oxatricyclo[5.2.1.0^{2,6}]deca-8-en-3-one, optically active bodies of these compounds, and the like.

According to the present invention, polycyclic lactams can be produced with high yield from the correspondent lactones.

The polycyclic lactams obtained by the production method of the present invention are useful as chemical raw materials and medical-agricultural drug intermediates, and can be suitably used as a production intermediate for the following compound (see, WO 2004/113295) which is one of anti-hepatitis C drugs (HCV drugs).

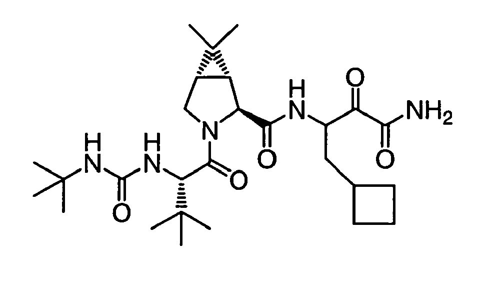

### EXAMPLES

The present invention will be illustrated further in detail based on examples below, but it is needless to say that the present invention is not limited to these examples.

### Example 1

### Synthesis Example of (1R,3S)-2,2-dimethyl-3-[(1,3-dihydro-1,3-dioxo-2H-isoindol-2-yl)methyl]cyclopropanecarboxylic acid

29.70 g (235 mmol) of (1R,5S)-6,6-dimethyl-3-oxabicyclo[3.1.0]hexan-2-one, 66.8 g of N,N-dimethylacetamide and 48.0 g (259 mmol) of phthalimide potassium were mixed and heated up to 155°C, then, stirred for 9 hours, to obtain a reaction solution containing potassium (1R,3S)-2,2-dimethyl-3-[(1,3-dihydro-1,3-dioxo-2H-isoindol-2-yl)methyl]cyclopropanecarboxylate. This solution was dropped at 15°C into mixed liquid of 67 g of water and 104 g of toluene. Next, 26.5 g of 35% hydrochloric acid was dropped, then, the mixture was heated up to 60°C, then, liquid partitioning was performed. As the organic layer, 213 g of a solution was obtained.

This solution contained 61.96 g (227 mmol) of (1R,3S)-2,2-dimethyl-3-[(1,3-dihydro-1,3-dioxo-2H-isoindol-2-yl)methyl]cyclopropanecarboxylic acid.

The yield with respect to (1R,5S)-6,6-dimethyl-3-oxabicyclo[3.1.0]hexan-2-one was 96.3%.

The quantitative determination of 2,2-dimethyl-3-[(1,3-dihydro-1,3-dioxo-2H-isoindol-2-yl)methyl]cyclopropanecarboxylic acid was effected by high performance liquid chromatography. As the column of high performance liquid chromatograph, SUMIPAX ODS D-210FF (4.6 mmφ × 150 mm, 3 µm)(manufactured by Sumika Chemical Analysis Service, Ltd.) was used.

The melting point of (1R,3S)-2,2-dimethyl-3-[(1,3-dihydro-1,3-dioxo-2H-isoindol-2-yl)methyl]cyclopropanecarboxylic acid was 159 to 160°C.

The measurement results of ¹H-NMR (CDCl₃) are shown below.
δ = 7.69 to 7.89 m (4H), 4.17 dd (1H), 4.00 dd (1H) ,1.54 to 1.66 m (2H), 1.41 s (3H), 1.18 s (3H)

### Example 2

### Synthesis Example of (1R,3S)-2,2-dimethyl-3-[(1,3-dihydro-1,3-dioxo-2H-isoindol-2-yl)methyl]cyclopropanecarboxylic acid

218 g (1.73 mol) of (1R,5S)-6,6-dimethyl-3-oxabicyclo[3.1.0]hexan-2-one, 1090 g of N,N-dimethylacetamide and 352 g (1.90 mol) of phthalimide potassium were mixed and heated up to 155°C, then, stirred for 17 hours to obtain a reaction solution containing potassium (1R,3S)-2,2-dimethyl-3-[(1,3-dihydro-1,3-dioxo-2H-isoindol-2-yl)methyl]cyclopropanecarboxylate. Next, the solvent was distilled off by concentration under reduced pressure, then, 763 g of toluene was added. Further, 693 g of 10% hydrochloric acid was dropped and mixed, then, liquid partitioning was performed. The resultant organic layer was concentrated under reduced pressure to distill of the solvent, then, 1275 g of methanol was added to obtain 2070 g of a solution containing 427 g (1.56 mol) of (1R,3S)-2,2-dimethyl-3-[(1,3-dihydro-1,3-dioxo-2H-isoindol-2-yl)methyl]cyclopropanecarboxylic acid.

The yield with respect to (1R,5S)-6,6-dimethyl-3-oxabicyclo[3.1.0]hexan-2-one was 90.5%.

### Example 3

### Synthesis Example of (1R,3S)-2,2-dimethyl-3-[(1,3-dihydro-1,3-dioxo-2H-isoindol-2-yl)methyl]cyclopropanecarboxylic acid

0.50 g (4.0 mmol) of (1R,5S)-6,6-dimethyl-3-oxabicyclo[3.1.0]hexan-2-one, 0.81 g of phthalimide potassium and 5.0 g of N-methyl-2-pyrrolidinone were mixed and heated up to 160°C, then, stirred for 24 hours to obtain a reaction solution containing potassium (1R,3S)-2,2-dimethyl-3-[(1,3-dihydro-1,3-dioxo-2H-isoindol-2-yl)methyl]cyclopropanecarboxylate. This reaction solution was cooled, then, 3.2 g of 5% hydrochloric acid was added, and extracted twice with 10 g of methyl tert-butyl ether to obtain a solution containing 0.84 g (3.1 mmol) of (1R,3S)-2,2-dimethyl-3-[(1,3-dihydro-1,3-dioxo-2H-isoindol-2-yl)methyl]cyclopropanecarboxylic acid.

The yield with respect to (1R,5S)-6,6-dimethyl-3-oxabicyclo[3.1.0]hexan-2-one was 77.5%.

### Example 4

### Synthesis Example of 2,2-dimethyl-3-[(1,3-dihydro-1,3-dioxo-2H-isoindol-2-yl)methyl]cyclopropanecarboxylic acid

12.0 g (95 mmol) of 6,6-dimethyl-3-oxabicyclo[3.1.0]hexan-2-one, 120 g of N,N-dimethylformamide and 17.6 g (95 mmol) of phthalimide potassium were mixed and stirred, and heated up to 155°C, then, reaction liquid containing potassium 2,2-dimethyl-3-[(1,3-dihydro-1,3-dioxo-2H-isoindol-2-yl)methyl]cyclopropanecarboxylate was obtained. The reaction liquid was cooled down to 25°C, then, 10.9 g of 35% hydrochloric acid was dropped, further, 634 g of water was dropped. This was cooled down to 0°C, then, the resultant crystal was filtrated. Then crystal was washed with water, then, dried under reduced pressure, to obtain 23.3 g of 2,2-dimethyl-3-[(1,3-dihydro-1,3-dioxo-2H-isoindol-2-yl)methyl]cyclopropanecarboxylic acid.

The yield with respect to 6,6-dimethyl-3-oxabicyclo[3.1.0]hexan-2-one was 84.5%.

The measurement results of ¹H-NMR (DMSO) of 2,2-dimethyl-3-[(1,3-dihydro-1,3-dioxo-2H-isoindol-2-yl)methyl]cyclopropanecarboxylic acid are shown below.
δ = 7.60 to 8.15 m (4H), 4.04 m (1H), 3.78 dd (1H), 1.48 to 1.56 m (2H), 1.26 s (3H), 1.09 s (3H)

### Example 5

### Synthesis Example of methyl (1R,3S)-2,2-dimethyl-3-[(1,3-dihydro-1,3-dioxo-2H-isoindol-2-yl)methyl]cyclopropanecarboxylate

52.7 kg of a toluene solution containing 13.1 kg (46.8 mol) of (1R,3S)-2,2-dimethyl-3-[(1,3-dihydro-1,3-dioxo-2H-isoindol-2-yl)methyl]cyclopropanecarboxylic acid obtained in the same manner as in Example 1 was concentrated under reduced pressure to distill off toluene. To this was added 26.1 kg of methanol and the temperature was controlled at 50°C, then, 6.8 kg of thionyl chloride was dropped and the mixture was stirred for 19 hours. To the reaction liquid was added 13.1 kg of toluene, then, the reaction liquid was dropped into a solution prepared by dissolving 4.9 kg of sodium hydrogen carbonate in 55.5 kg of water. Further, 52.3 kg of toluene was added, then, pH was adjusted to around 7 using 12.7 kg of a 28% sodium hydroxide aqueous solution. This mixed liquid was partitioned, and the resultant organic layer was concentrated under reduced pressure to distill off toluene, then, 105 kg of methanol was added to obtain 125 kg of a solution containing 13.7 k g (47.6 mol) of methyl (1R,3S)-2,2-dimethyl-3-[(1,3-dihydro-1,3-dioxo-2H-isoindol-2-yl)methyl]cyclopropanecarboxylate.

The yield with respect to (1R,3S)-2,2-dimethyl-3-[(1,3-dihydro-1,3-dioxo-2H-isoindol-2-yl)methyl]cyclopropanecarboxylic acid was 99.2%.

The quantitative determination of methyl (1R,3S)-2,2-dimethyl-3-[(1,3-dihydro-1,3-dioxo-2H-isoindol-2-yl)methyl]cyclopropanecarboxylate was effected by high performance liquid chromatography. As the column of high performance liquid chromatograph, SUMIPAX ODS D-210FF (4.6 mmφ × 150 mm, 3 µm)(manufactured by Sumika Chemical Analysis Service, Ltd.) was used.

The melting point of methyl (1R,3S)-2,2-dimethyl-3-[(1,3-dihydro-1,3-dioxo-2H-isoindol-2-yl)methyl]cyclopropanecarboxylate was 85 to 86°C.

The measurement results of ¹H-NMR (CDCl₃) are shown below. δ = 7.68 to 7.88 m (4H), 4.17 dd (1H), 3.97 dd (1H), 3.71 s (3H), 1.62 d (1H), 1.44 to 1.59 m (1H), 1.37 s (3H), 1.15 s (3H)

### Example 6

### Synthesis Example of methyl 2,2-dimethyl-3-[(1,3-dihydro-1,3-dioxo-2H-isoindol-2-yl)methyl]cyclopropanecarboxylate

30.3 g (110 mmol) of 2,2-dimethyl-3-[(1,3-dihydro-1,3-dioxo-2H-isoindol-2-yl)methyl]cyclopropanecarboxylic acid and 130 g of methanol were mixed and the temperature was controlled at 50°C, then, 47.0 g of thionyl chloride was dropped and the mixture was stirred for 5 hours. The reaction liquid was concentrated under reduced pressure to distill off the solvent, then, 130 g of methanol was added, and concentrated under reduced pressure again to distill off the solvent. The resultant solid was dried under reduced pressure to obtain 31.5 g of methyl 2,2-dimethyl-3-[(1,3-dihydro-1,3-dioxo-2H-isoindol-2-yl)methyl]cyclopropanecarboxylate.

The yield with respect to (1R,3S)-2,2-dimethyl-3-[(1,3-dihydro-1,3-dioxo-2H-isoindol-2-yl)methyl]cyclopropanecarboxylic acid was 99%.

The measurement results of ¹H-NMR (DMSO) of methyl 2,2-dimethyl-3-[(1,3-dihydro-1,3-dioxo-2H-isoindol-2-yl)methyl]cyclopropanecarboxylate are shown below.
δ = 7.82 to 7.90 m (4H), 4.02 dd (1H), 3.77 dd (1H), 3.62 s (3H), 1.65 d (1H), 1.60 to 1.51 m (1H), 1.25 s (3H), 1.10 s (3H)

### Example 7

### Synthesis Example of (1R-cis)-6,6-dimethyl-3-azabicyclo[3.1.0]hexan-2-one

125 kg of a methanol solution containing 13.6 kg (47.4 mol) of methyl (1R,3S)-2,2-dimethyl-3-[(1,3-dihydro-1,3-dioxo-2H-isoindol-2-yl)methyl]cyclopropanecarboxylate obtained in Example 5 was mixed with 5.4 kg (64 mol) of a 60% aqueous solution of hydrazine monohydrate, and the temperature was raised to 65°C, then, the mixture was stirred for 25 hours. The reaction liquid was cooled down to 25°C, 1.6 kg of 78% sulfuric acid was added, and the solvent was distilled off by concentration under reduced pressure. Further, 54.5 kg of toluene was added, and again, the solvent was distilled off by concentration under reduced pressure. To this was added 47.7 kg of toluene, then, the deposited crystal was filtrated. From the filtrate, the solvent was distilled off by concentration under reduced pressure to obtain 36 kg of a solution containing 5.94 kg (47 mol) of (1R-cis)-6,6-dimethyl-3-azabicyclo[3.1.0]hexan-2-one.

The yield with respect to methyl (1R,3S)-2,2-dimethyl-3-[(1,3-dihydro-1,3-dioxo-2H-isoindol-2-yl)methyl]cyclopropanecarboxylate was 100%.

The quantitative determination of (1R-cis)-6,6-dimethyl-3-azabicyclo[3.1.0]hexan-2-one was effected by high performance liquid chromatography. As the column,
SUMIPAX ODS D-210FF (4.6 mmφ × 150 mm, 3 µm)(manufactured by Sumika Chemical Analysis Service, Ltd.) was used.

### Example 8

### Synthesis Example of 6,6-dimethyl-3-azabicyclo[3.1.0]hexan-2-one

28.0 g (97 mmol) of methyl 2,2-dimethyl-3-[(1,3-dihydro-1,3-dioxo-2H-isoindol-2-yl)methyl]cyclopropanecarboxylate obtained in Example 6 and 222 g of methanol were mixed and to this was added 5.9 g (118 mmol) of hydrazine monohydrate, and the temperature was controlled at 78°C, then, the mixture was stirred for 21 hours. This reaction liquid was concentrated under reduced pressure to distill off the solvent. To the resultant solid was added 98 g of methyl tert-butyl ether and the mixture was stirred, then, insoluble materials were filtrated. The filtrate was concentrated under reduced pressure to distill of the solvent, and the resultant solid was dried under reduced pressure to obtain 11.1 g of 6,6-dimethyl-3-azabicyclo[3.1.0]hexan-2-one.

The yield with respect to methyl 2,2-dimethyl-3-[(1,3-dihydro-1,3-dioxo-2H-isoindol-2-yl)methyl]cyclopropanecarboxylate was 99%.

### Industrial Applicability

The lactams obtained by the production method of the present invention can be used suitably as chemical raw materials and medical-agricultural drug intermediates.

## Claims

1. A method of producing lactams of the following formula (1) comprising the following three steps A to C: 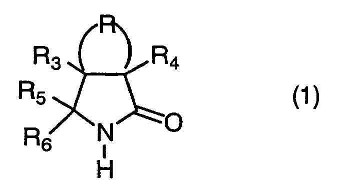 [in the formula (1), when R₃ and R₄ are both a hydrogen atom, R represents a substituted methylene group or an optionally substituted polymethylene group having 2 to 4 carbon atoms, when at least one of R₃ and R₄ is a substituent other than a hydrogen atom, R represents an optionally substituted methylene group or an optionally substituted polymethylene group having 2 to 4 carbon atoms, and one or no-mutually-adjacent two methylene groups constituting the polymethylene group may be substituted by an oxygen atom, one or two ethylene groups constituting the polymethylene group may be substituted by a vinylene group, no-mutually-adjacent two methylene groups constituting the polymethylene group may be mutually connected via an oxygen atom, methylene group, ethylene group or vinylene group,
R₃, R₄, R₅ and R₆ represent each independently a hydrogen atom, halogen atom, cyano group, optionally substituted alkyl group having 1 to 10 carbon atoms, optionally substituted alkenyl group having 2 to 10 carbon atoms, optionally substituted aryl group having 6 to 20 carbon atoms, optionally substituted amino group, -ORₐ group, -SR_{b} group or -COOR_{c} group, Rₐ and R_{b} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms, and R_{c} represents a hydrogen atom, alkyl group having 1 to 10 carbon atoms or aralkyl group having 7 to 20 carbon atoms.]
(Step A)
a step of reacting lactones of the following formula (2): 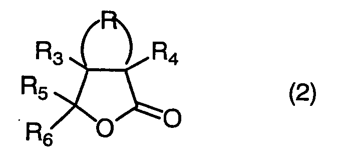 [in the formula (2), R and R₃ to R₆ represent the same meanings as described above.]
and imides of any of the following formulae (3a), (3b) and (3c): 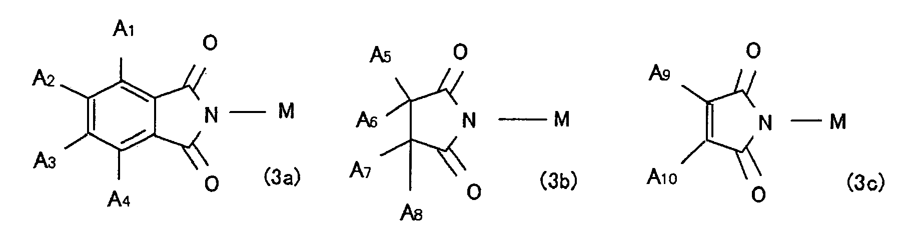 [in the formulae (3a), (3b) and (3c), A₁, A₂, A₃, A₄, A₅, A₆, A₇, A₈, A₉ and A₁₀ represent each independently a hydrogen atom, halogen atom, nitro group, alkyl group having 1 to 6 carbon atoms, alkenyl group having 2 to 4 carbon atoms, optionally substituted aryl group having 6 to 20 carbon atoms, optionally substituted acyl group having 2 to 4 carbon atoms or -OR_{d}, R_{d} represents a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms, and M represents a hydrogen atom or alkali metal atom.]
to produce the correspondent carboxylic acids of the following formulae (4a), (4b) and (4c): 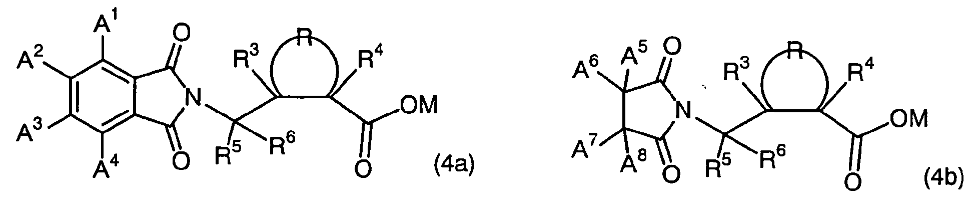 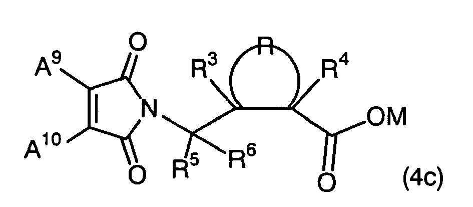 [in the formulae (4a), (4b) and (4c), R, R₃, R₄, R₅, R₆, A₁, A₂, A₃, A₄, A₅, A₆, A₇, A₈, A₉, A₁₀ and M represent the same meanings as described above.]
(Step B)
a step of esterifying the carboxylic acids of any of the formulae (4a), (4b) and (4c) obtained in the step A to produce the correspondent esters of the following formulae (5a), (5b) and (5c): 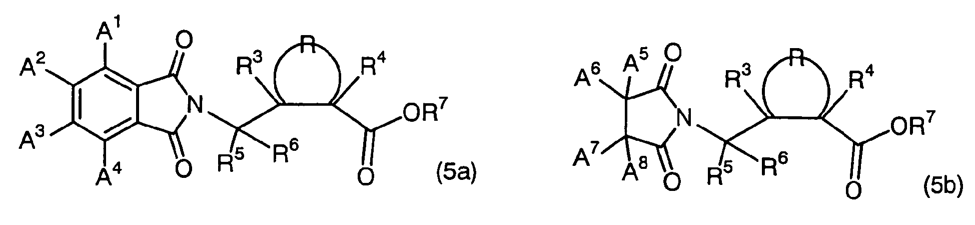 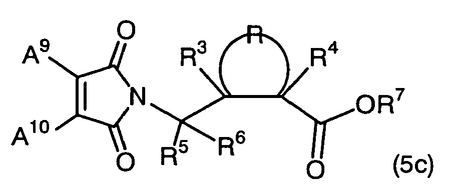 [in the formulae (5a), (5b) and (5c), R, R₃, R₄, R₅, R₆, A₁, A₂, A₃, A₄, A₅, A₆, A₇, A₈, A₉ and A₁₀ represent the same meanings as described above, and R₇ represents an alkyl group having 1 to 10 carbon atoms, alkenyl group having 2 to 10 carbon atoms or aralkyl group having 7 to 20 carbon atoms.]
(Step C)
a step of reacting the esters of any of the formulae (5a), (5b) and (5c) obtained in the step B and amines to produce lactams of the formula (1).

2. The production method according to Claim 1
wherein the lactones of the formula (2) and the imides of any of the formulae (3a), (3b) and (3c) are reacted in an amide solvent of the following formula (6): 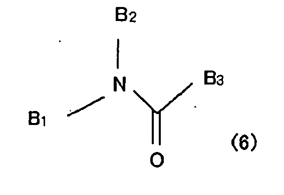 [in the formula (6), B₁ and B₂ represent each independently a hydrogen atom or alkyl group having 1 to 5 carbon atoms, B₃ represents a hydrogen atom, alkyl group having 1 to 10 carbon atoms or aryl group having 6 to 20 carbon atoms, and B₂ and B₃ may be connected to form a cyclic structure together with an atom to which they are connected.].

3. The production method according to Claim 2
wherein the amide solvent of the formula (6) is N,N-dimethylacetamide.

4. The production method according to any one of Claims 1 to 3 wherein the imides are imides of the formula (3a).

5. The production method according to any one of Claims 1 to 4 wherein the imides of the formula (3a) are phthalimide potassium.

6. The production method according to any one of Claims 1 to 5 wherein the carboxylic acids of any of the formulae (4a), (4b) and (4c) and alcohols of the following formula (7)
R₇-OH (7)
(wherein, R₇ represents an alkyl group having 1 to 10 carbon atoms, alkenyl group having 2 to 10 carbon atoms or aralkyl group having 7 to 20 carbon atoms.) are reacted to produce esters of any of the formulae (5a), (5b) and (5c), and the resultant esters of any of the formulae (5a), (5b) and (5c) and amines of the following formula (8):
R₈-NH₂ (8)
(wherein, R₈ represents an optionally substituted amino group or alkyl group having 1 to 5 carbon atoms optionally substituted with an amino group.) are reacted to produce lactams of the formula (1).

7. The production method according to any one of Claims 1 to 6 wherein R₅ and R₆ represent a hydrogen atom.

8. The production method according to any one of Claims 1 to 7 wherein R₃ and R₄ represent a hydrogen atom.

9. The production method according to any one of Claims 1 to 8 wherein R is a group selected from the following group:
- (CH₂)₂-, -(CH₂)₃-, -(CH₂)₄-, (CH3)₂C<, (C1)₂C<, (F)₂C<, >CH(CO₂C₂H₅),

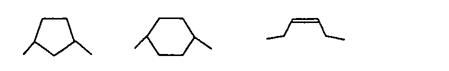

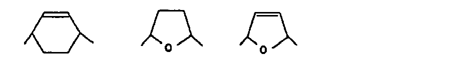

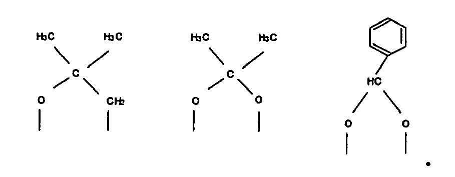

10. The production method according to any one of Claims 1 to 9 wherein R is a group selected from the following group:
-(CH₂)₂-, -(CH₂)₃-, -(CH₂)₄-, (CH₃)₂C<.

11. The production method according to any one of Claims 1 to 10 wherein R is a (CH₃)₂C< group.

12. Carboxylic acids of the following formula (4a): 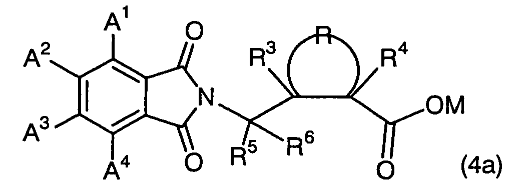 [in the formula (4a), R, R₃ to R₆, A₁ to A₄ and M represent the same meanings as described above.].

13. The carboxylic acids according to Claim 12
wherein, in the formula (4a), R represents a group selected from the group consisting of -(CH₂)₂-,-(CH₂)₃-, -(CH₂)₄- and (CH₃)₂C<, and R₃, R₄, R₅, R₆, A₁, A₂, A₃ and A₄ represent a hydrogen atom.

14. 2,2-dimethyl-3-[(1,3-dihydro-1,3-dioxo-2H-isoindol-2-yl)methyl]-cyclopropanecarboxylic acid.

15. A potassium salt of 2,2-dimethyl-3-[(1,3-dihydro-1,3-dioxo-2H-isoindol-2-yl)methyl]-cyclopropanecarboxylic acid.

16. (1R,3S)-2,2-dimethyl-3-[(1,3-dihydro-1,3-dioxo-2H-isoindol-2-yl)methyl]-cyclopropanecarboxylic acid.

17. A potassium salt of (1R,3S)-2,2-dimethyl-3-[(1,3-dihydro-1,3-dioxo-2H-isoindol-2-yl)methyl]-cyclopropanecarboxylic acid.

18. Esters of the following formula (5a): 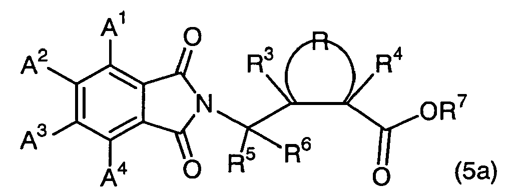 [in the formula (5a), R, R₃ to R₇ and A₁ to A₄ represent the same meanings as described above.].

19. The esters according to Claim 18 wherein, in the formula (5a), R represents a group selected from the group consisting of -(CH₂)₂-, -(CH₂)₃-, -(CH₂)₄-and (CH₃)₂C<, and R₃, R₄, R₅, R₆, A₁, A₂, A₃ and A₄ represent a hydrogen atom.

20. The esters according to Claim 18 or 19 wherein R₇ in the formula (5a) is an alkyl group having 1 to 4 carbon atoms.

21. 2,2-dimethyl-3-[(1,3-dihydro-1,3-dioxo-2H-isoindol-2-yl)methyl]-cyclopropanecarboxylates.

22. Methyl 2,2-dimethyl-3-[(1,3-dihydro-1,3-dioxo-2H-isoindol-2-yl)methyl]-cyclopropanecarboxylate.

23. Methyl (1R,3S)-2,2-dimethyl-3-[(1,3-dihydro-1,3-dioxo-2H-isoindol-2-yl)methyl]-cyclopropanecarboxylate.
